Europäisches Patentamt

(19) European Patent Office

Office européen des brevets

(11) EP 1 276 763 B1

(12) EUROPEAN PATENT SPECIFICATION

(45) Date of publication and mention
of the grant of the patent:
**25.02.2004 Bulletin 2004/09**

(51) Int Cl.[7]: **C07K 14/41**, A23G 9/02,
A23L 3/3526

(21) Application number: **01919437.2**

(22) Date of filing: **06.04.2001**

(86) International application number:
**PCT/EP2001/003927**

(87) International publication number:
**WO 2001/083534 (08.11.2001 Gazette 2001/45)**

(54) **ANTI-FREEZE PROTEINS, THEIR PRODUCTION AND USE**

ANTI-GEFRIER PROTEINE, DEREN HERSTELLUNG UND VERWENDUNG

PROTEINES ANTIREFRIGERANTES, PRODUCTION ET UTILISATION DE CELLES-CI

(84) Designated Contracting States:
**AT BE CH CY DE DK ES FI FR GB GR IE IT LI LU MC NL PT SE TR**

(30) Priority: **27.04.2000 GB 0010314**

(43) Date of publication of application:
**22.01.2003 Bulletin 2003/04**

(73) Proprietors:
• **UNILEVER PLC**
**London EC4P 4BQ (GB)**
Designated Contracting States:
**CY GB IE**
• **UNILEVER N.V.**
**3013 AL Rotterdam (NL)**
Designated Contracting States:
**AT BE CH LI DE DK ES FI FR GR IT LU MC NL PT SE TR**

(72) Inventors:
• **BERRY, Mark John, Unilever Research Colworth**
**Bedford, Bedfordshire MK44 11Q (GB)**
• **DOUCET, Charlotte Juliette, University of York**
**Heslington, Yorkshire YO1 5YM (GB)**
• **LUNDHEIM, Rolv Sigmund**
**Thonnig Owesens GT,18,N-N-7044 Trondheim (NO)**
• **SEVILLA, Marie-Pierre**
**31520 Ramonville saint agne (FR)**
• **WHITEMAN, Sally-Anne,**
**Unilever Research Colworth**
**Bedford, Bedfordshire MK44 1LQ (GB)**

(74) Representative: **Hugot, Alain et al**
**Unilever plc,**
**Patent Division,**
**Colworth House**
**Sharnbrook, Bedford MK44 1LQ (GB)**

(56) References cited:
**WO-A-98/04148          WO-A-99/37673**

## Description

Field of the Invention

The present invention relates to antifreeze proteins, and to processes for their production and use. More specifically, it relates to obtaining antifreeze proteins from an organism not previously used for the purpose; to the novel antifreeze proteins derived therefrom; and to the use of such proteins in controlling freezing processes, especially in the production of frozen food; and the foods thereby obtained.

Background to the Invention

So-called 'antifreeze proteins' (AFPs) have the property of modifying the growth of ice crystals. They differ in their action from simpler ionic antifreeze agents such as common salt. For example, aqueous AFP solutions typically have a freezing point that is lower than their melting point (hysteresis). They inhibit recrystallisation. They seem to assist organisms to survive in temperatures around the freezing point of water, and are accordingly found in several different types of organism. Their properties give them a range of potential uses: in particular in foods that are eaten while frozen, by inhibiting recrystallisation and maintaining a smooth texture. In foods that are frozen only for preservation, AFPs may inhibit recrystallisation during freezing, storage, transport, and thawing, thus preserving food texture by reducing cellular damage and also minimising the loss of nutrients by reducing drip (see Griffith, M. and Vanya Ewart, K. *Biotechnology Advances*, 13, pp 375-402).

Various sources of AFPs are known: the most widely reported are fish and plants. Some bacteria exhibit antifreeze properties (see Griffith et al, supra, p 382): in a few cases, the isolation of antifreeze proteins from bacteria has been reported (see for example: Xu H, Griffith M, Patten CL, et al., Can J Microbiol 44: (1) 64-73 Jan 1998).

Given the advantages to an organism of resistance to freezing, it may be considered surprising that antifreeze proteins are not more widely distributed in nature and easier to find.

One drawback to using novel AFPs in frozen food is the need to be sure that such AFPs are safe and acceptable to the consumer. Consumers reasonably require assurance that what they are eating is safe. They also may show strong preferences for or against particular types of material: for example, for 'natural' or 'organic' materials, and against 'additives', 'chemicals' or 'genetically modified materials - (GM)'. Food manufacturers ignore such preferences at their peril.

There is accordingly a demand for effective AFPs which are, or can be seen as, 'natural'. If such materials are derived from sources which have a history of consumption by human beings, this gives a reasonable presumption that they are more likely to be safe. This can reduce the need for extensive, and expensive, toxicity testing.

It has previously been reported that some lichens contain antifreeze proteins. In particular, AFPs from *Alectoria nigricans, Caloplaca regalis, Himantormia lugubris, Hypogymnia physodes, Parmelia subrudecta, Ramalina farinacea, Stereocaulon glabrum, Umbilicaria antarctica*, and *Usnea subfloridana* are known [WO 98/04148]. Furthermore, such AFPs have been proposed for use in frozen confectionery [WO 98/04148].

In WO 99/37673, an AFP which can be derived from lichen having an apparent molecular weight of from 20 to 28 kDa and an N-terminal amino acid sequence which shows at least 80% overlap with A-P-A-V-V-M-G-D-A-E-S-F-G-A-I-A-H-G-G-L is described.

There are, however, a number of drawbacks to using AFPs obtained from lichen as ingredients in food products. Not all lichens show AFP activity. Of those that do, many have not regularly been consumed by humans. Lichens are usually impracticable to propagate on a commercial scale, and many are only available in the environment in very small quantities. Thus it is not obvious to look closely among lichens as a source for AFPs. Nevertheless such research was undertaken, leading to the present invention.

In the hope of finding suitable new AFPs, four candidate lichens growing wild in Norway were selected for testing. These were *Cetraria islandica, Nephroma arcticum* (Nephromatoceae Peltigerales family), *Umbilicaria hyperborea* (Umbilicariaceae family) and *Platismatia glauca*. These are among the many lichens to be found growing in large quantities in Norwegian forests: each was readily collected in amounts of 100 gram or more.

*Cetraria islandica* is the lichen species most frequently used as food. It has been called "Brodmose" ("bread moss"). During the 18th and 19th centuries, the Norwegian government encouraged people to use more lichen in the household. *C. islandica* was mostly used when the crop froze, and during wars. It has been used as recently as World War II. *Umbilicaria sp.* has also been used as food, and has the name "geitbrod" ("goat bread"). *Cetraria nivalis, Cladonia sp.* and *Peltigera aphtosa* have also been used as food.

*Nephroma arcticum* has also been used in food, albeit not so frequently as *C. islandica*; it has also traditionally been used as medicine against skin diseases. *N. arcticum* is a foliose lichen with relatively large and thick thalli. The upper surface is yellow-green or green when wet, and straw-coloured or grey-green when dry. The lower surface is brown and white towards the edges.

This species grows mainly on shaded rocks and on the ground. It is a boreal/alpine species with its main distribution in the low alpine environment. It is a circumpolar species found in North America, Asia (including Japan), and Europe. In Britain it is very rare. It is found all over Norway with the exception of the West Coast of South Norway, though often little is found in each locality. So far as is known, its abundance has not until now been investigated in detail. It is not a protected species in Norway, nor likely to become one.

Since the thalli of *Nephroma arcticum* are large and thick they are relatively easy to rinse. Therefore it is easy to obtain clean samples. Also, in some localities the lichen covers much of the ground. Therefore, when such a locality is found, the lichen can be collected rapidly.

Summary of the Invention

The invention provides antifreeze proteins (AFPs) which can be derived from the lichen *Nephroma arcticum*, particularly those having an amino acid sequence from the N-terminus of L-V-I-G-S-T-A-Q(E)-N-F-G-V-V(S)-A-A-A-T.

Also provided are AFPs comprising an amino acid sequence which has a high degree of similarity with the above sequence or modified versions thereof.

Nucleic sequences encoding such proteins and vectors comprising such sequences are also encompassed by the invention.

The invention further provides a process for preparing AFPs which comprises extracting an AFP protein from the lichen *Nephroma arcticum*, their use in food processing and food compositions containing them.

As used herein, the term 'antifreeze protein' (AFP) means a protein, which may be a modified protein, that is capable of inhibiting the growth of ice crystals (see, for example, US 5,118,792). Accordingly, 'AFP activity' is the ability of a protein to inhibit the growth of ice crystals. This may be demonstrated, for example, using a 'splat assay' (see, for example, Smallwood et al, Biochem. J. 340, 385-391).

A protein that is 'capable of being derived from' a specified organism is a protein that is encoded by a gene that is naturally present in that organism; the protein may be derived either by extraction from the organism itself or by heterologous expression of a nucleic acid sequence encoding the protein in a suitable host.

Detailed Description of the Invention

The present invention is based on the discovery of a protein with powerful AFP activity in a known human food source. Extracting this protein from the source gives an effective AFP that is likely to be safe, because it comes from a food source, and which may be considered 'natural' by consumers.

Specifically, the invention comprises novel proteins obtainable from *Nephroma arcticum* having antifreeze properties. One major antifreeze protein has so far been identified by the inventors and its sequence has been partly determined. The invention also encompasses other proteins that may be contributory to the antifreeze activity in this lichen species.

The major AFP isolated from *Nephroma arcticum* has an apparent molecular weight, as judged by SDS-polyacrylamide gel electrophoresis, of around 29 kDa, (although given the limitations of the technique there is a likely margin of error of +/- 4 kDa on this value). The N-terminal amino acid sequence of this proteins has been determined to be:

L-V-I-G-S-T-A-Q(E)-N-F-G-V-V(S)-A-A-A-T

There appears to be some sequence heterogeneity at positions 8 (major form Q with E as a minor variant) and 13 (major form V with S as a minor variant)as indicated. A similarity search against peptide sequence databases did not identify any known polypeptides with significant homology to this sequence.

The invention specifically includes this protein, whether it be obtained directly from *Nephroma arcticum*, or by expression of a DNA sequence encoding it in a genetically modified organism.

Also included within the scope of the invention are proteins,from whatever source, which exhibit a high degree of sequence similarity with the above listed sequence. For the purposes of the invention, all proteins that exhibit AFP activity and which have an amino acid sequence part of which shows at least 80% overlap with the above sequence are included within the scope. More preferred is an overlap of at least 90%, most preferred at least 95%; for example, sequences which differ from the above by no more than 1 or 2 residues in the appropriate part of their sequences.

For the purpose of the invention, the degree of overlap of two (partial) amino acid sequences can be calculated as follows:

(a) the two sequences are aligned and the number of residues that are identical and appear in the same order is counted (x);
(b) every change, deletion or insertion of an amino acid is counted (1 point) and the total of such changes, deletions or insertions is calculated (y);

(c) the degree of overlap is calculated as
x.100%/(x+y)

It will be appreciated that where the similar protein has an amino acid sequence having an N-terminal extension, the amino acid sequences starting from the N-terminal end may not in themselves be at least 80% similar. Such proteins nevertheless form part of the invention provided that at least part of the amino acid sequence shows at least 80% overlap with the above sequence.

Also included within the scope of the invention is any modified version of any of the above-described proteins, provided that the modification does not significantly affect the AFP activity (as judged, for example in a splat assay)of said modified protein. For example, glycosylated proteins are included.

The invention further comprises nucleic acid sequences encoding the novel proteins of the invention. The DNA sequence of the major AFP that has already been partially sequenced can readily be cloned and characterised using methods well known in the art; a suitable method is outlined in Example 11. DNA sequences encoding other AFPs that may be present can similarly be derived once partial protein sequences are obtained.

Vectors comprising a DNA sequence encoding an AFP according to the invention are also included within the scope of the invention.

For some applications, it may be possible to collect sufficient *Nephroma arcticum* from the wild without harming the environment. Indeed, the inventors note that some lichens are already harvested commercially for use as reindeer food. The invention therefore provides a process for the production of a material containing an AFP, said process comprising collecting the lichen, *Nephroma arcticum*, from the wild, and recovering from it an extract that exhibits AFP activity.

The applicability of this process on an industrial scale is clearly dependent on the availability of the lichen and an important feature of the invention is that the inventors have surveyed the mountains of central Norway and found that *Nephroma arcticum* is abundant and can therefore be collected in sufficient quantity to use as an additive in processed foods. The inventors have found new, rich localities of this species and they have concluded that *N. arcticum* is very common in these mountains. It is interesting to note that data from Norwegian museum herbaria are scarce and can give the impression that this species is not very abundant. This reflects a lack of investigation more than the real distribution of the lichen. However, a problem with the mountain localities is that in these areas the lichen has a growth form that makes it difficult to collect. It grows very closely attached to soil and moss and each thallus is smaller than in lowland localities. Therefore, the best strategy for collection is probably to search for rich localities below the timber line.

Methods for preparing crude protein extracts from microorganisms and for the partial or complete purification of proteins with specific activities from such extracts are very well known in the art. A method suitable for isolation of a very pure preparation of a major AFP from *N. arcticum*, using a combination of ion exchange, affinity chromatography and gel filtration, is given in Example 9. For application in food preparation, such a high degree of purification would not be necessary. However, partial purification, for example to remove proteases that might otherwise tend to degrade the AFP over time, might be desirable.

When it is desired to use the AFP in a food product, the extraction process must be adapted to use only non-toxic chemicals. Such a process is described in example 6, although other extraction processes could also be used. It is important to note that before using any such AFP preparation in frozen foods it may be necessary to confirm that the preparation is indeed non-toxic. Specialised toxicological laboratories exist that undertake such work using techniques that are well known to experts in the field.

The invention also provides a process for producing a material with AFP activity, said process comprising culturing an organism containing a DNA sequence coding for a novel protein according to the invention, under the control of suitable gene regulatory elements and under conditions in which the protein is synthesised, and recovering from the culture an extract that exhibits AFP activity. This organism may be one of the component organisms of *Nephroma arcticum*, in which the gene encoding the AFP occurs naturally, or it may be a different organism that has been genetically modified to produce the AFP.

Lichen consist of two or more organisms living in symbiosis. The mycobiont is a fungus and the photobiont is either an alga or a cyanobacterium. In some cases, including *Nehproma arcticum*, the fungus lives in symbiosis with both algae and cyanobacteria at the same time. The photobiont provides energy from photosynthesis.

It is possible to culture the component organisms of a lichen such as *Nehproma arcticum*. In particular, it is possible to culture the individual component organisms: a culture of the fungal partner can be produced, a culture of the algal partner can be produced, and a culture of the bacterial partner can be produced. The culture that produces the most AFP can be determined by using the standard AFP assay. Such cultures could then be used as a commercial source of AFP. This approach would have the advantage that, on the one hand it would not raise environmental issues, since *N. arcticum* would not be removed from the wild, and on the other hand GM-technology is not involved. This is potentially important because some consumers do not want their food to contain additives or ingredients that have been produced by genetic modification technology. It is taught how to culture the component organisms in Example 12.

An alternative strategy would be to culture a genetically transformed organism comprising a DNA sequence coding for an anti-freeze protein according to the invention, the sequence being under control of a gene promoter adapted to cause it to produce the antifreeze protein in the transformed organism. The DNA sequence coding for the antifreeze protein may be inserted into a suitable expression vector containing the necessary elements for transcription and translation into the desired protein under appropriate conditions, including proper orientation of the sequence, correct reading frame, and suitable targeting and expression sequences. Methods for making suitable vectors, and for using them to transform many different types of organism, are well understood in the art.

In principle, any organism may be modified to produce the desired protein in this way, for example, bacteria, yeasts, plants, or plant, insect or animal cells. Bacteria, yeasts and plants or plant cell systems are generally preferred. Genetically transformed organisms suitable for carrying out the process of the invention also form a further aspect of the invention.

Advantages of using a genetically transformed organism to produce the AFP could include easier handling and faster growth of the organism, as well as potentially much greater yields. Extraction and partial or complete purification might also be easier, especially if it is arranged that the AFP is secreted into the culture medium. An additional advantage of secretion is that with some host organisms, at least, the levels of protease accumulating in the medium are usually low, so that the stability of the AFP might be enhanced.

Methods suitable for preparing a protein-containing extract from such cultured organisms are well known in the art; steps suitable for partial or complete refinement of the AFP from such an extract would be similar to those used in extracting it from *the N. arcticum* extract itself, as described above.

It is also possible to provide suitable genetically transformed organisms, adapted to produce the AFPs of the invention, which are themselves useful on account of their increased frost resistance. In particular this applies to plants: which may be cereals such as wheat or maize; or dicotyledons such as soya, tomato or lettuce. Such plants also form part of our invention.

AFPs according to the invention can conveniently be used in several products, preferably in food products that are frozen or intended for freezing. Examples of such food products are: frozen food products such as vegetables, sauces, soups, snacks, dairy products and frozen confectionery, under which term we include sorbet, water-ice, granites, frozen fruit purees and milk-containing frozen confections such as ice-cream, frozen yoghurt or custards, sherbet and ice-milk. Preferred food products are frozen vegetables and frozen confectionery, e.g. ice-cream, water-ice. If dry-mixes or concentrates are used, the concentration may be higher in order to ensure that the level in the final frozen product is within the desired range.

It is possible to determine a suitable amount of an AFP-containing solution to add to a food-stuff by using a re-crystallisation inhibition (RI) assay as described, for example, in Example 7. The RI assay is quantitative but it is also time-consuming and requires special equipment. An assay for measuring ice re-crystallisation inhibition has previously been described by Jarman et al. (WO 99/37782). For most applications, the inventors have found that preferred levels of AFP are 0.00005 - 0.3%, particularly 0.0001 - 0.2% by weight of final product.

In preparing a food product it is not necessary to add the AFP in a highly purified form: it may be added as a composition containing the AFP, e.g. an extract of an organism producing the AFP. As discussed above, however, partial purification may be advantageous, for example, to remove proteases.

Frozen confectionery according to the invention can be produced by any suitable method known to the art. Preferably all ingredients of the formulation are fully mixed together at or above ambient temperature before freezing. The level of solids in the frozen confection (e.g., sugar, fat, flavoring) is suitably adjusted to be at least 3% and normally in the range 10 to 70%, particularly 40 to 70%, by weight of the final product.

The following examples are provided by way of illustration only:

## EXAMPLE 1

Collection of lichen and shipment to laboratories for investigation

The samples of lichen used in the examples were collected from a sub-alpine environment.

Selected lichens were picked by hand and kept in paper bags during transportation to the laboratory. At the laboratory the paper bags were put in a refrigerated room at +5°C for storage prior to rinsing.

The lichens were first rinsed under running tap water. After this the lichen was rinsed and sorted by hand when wet. After rinsing the lichens were flushed with liquid nitrogen and transferred to a freezer (-80°C) where they kept in paper bags inside cardboard boxes until shipment to the UK in Styrofoam containers with dry ice. All lichen were rinsed and frozen in liquid nitrogen within 36 hours after collection.

**EXAMPLE 2**

Assaying for AFP activity in two edible lichens (*Nephroma arcticum* and *Cetraria islandica*).

**Homogenisation of Lichen tissue:**

Frozen tissue, which had been stored at -80°C, was ground to a fine powder in liquid nitrogen in a pestle and mortar that had been pre-cooled at -20°C. The powder was transferred to a fresh pestle and mortar and homogenised further in an equal volume of buffer A (0.2M Tris; 10mM EDTA; 20mM ascorbate; pH 7.8; 30% sucrose w/w). The homogenate was centrifuged (Jouan 14 bench-top centrifuge) at 14,000 rpm for 4 minutes and the supernatant kept on ice prior to further use.

**Testing for AFP activity using the splat assay:**

AFP activity was determined using a modified "splat assay" (Smallwood et al, Isolation and characterisation of a novel antifreeze protein from carrot (Daucus carota). Biochem. J. **340,** 385-391). 4μl of the solution under investigation was transferred onto a clean, appropriately labelled, 13mm circular cover-slip. A second cover-slip was placed on top of the drop of solution and the two pressed together between finger and thumb to form a sandwich. The sandwich was dropped into a bath of heptane held at -80°C in a box of dry ice. When all sandwiches had been prepared, they were transferred from the -80°C heptane bath to the viewing chamber containing heptane held at -6°C using forceps pre-cooled in the dry ice. Upon transfer to -6°C, sandwiches could be seen to change from a transparent to an opaque appearance. Ice crystals were viewed using a 20x objective on an Optiphot microscope (Nikon) and images recorded after 60 minutes incubation at -6°C using a video camera and an image analysis system (LUCIA, Nikon).
For semi-quantitative analysis, serial 1:2 dilutions of supernatant were assayed to determine the lowest dilution at which reduced ice-crystal size could be detected. The Bio-Rad protein assay was used according to manufacturer's instructions with a standard curve of BSA to determine the protein content of the supernatant.

**Results:**

Extracts of *Nephroma arcticum* and *Cetraria islandica* were prepared and tested for AFP activity. *Nephroma arcticum* tested positive for AFP activity. *Cetraria islandica* tested negative for AFP activity. *Nephroma arcticum* showed significant AFP activity: serial 1:2 dilutions of the Nephroma arcticum sample showed that the AFP activity was still detectable at total protein levels of 0.1mg/ml.

**EXAMPLE 3**

Assaying for AFP activity in three abundant Lichens (*Nephroma arcticum, Umbilicaria hyperborea,* and *Plastismatia glauca*).

**Protein extraction:**

The three lichens, *Nephroma arcticum* (Nephromatoceae Peltigerales family), *Umbilicaria hyperborea* (Umbilicariaceae family) and *Platismatia glauca* were harvested during the winter in Norway and were stored at -80°C.
The lichen tissues were dropped in liquid nitrogen and immediately ground manually in separate mortars while adding regularly liquid nitrogen.
The crude protein extracts were prepared by stirring the ground lichen tissues (250g of *Nephroma arcticum*, 128g of *Umbilicaria hyperborea* and 100g of *Platismatia glauca)* with ice cold 50mM Tris/HCl 'Extraction buffer' (750ml, 550ml and 550ml respectively) in glass beakers for 30 minutes at 4°C. One litre 'Extraction buffer' contained 1 bottle of General use protease inhibitor cocktail from Sigma. The general use protease inhibitor cocktail contained 4-(2-aminoethyl) benzenesulfonyl fluoride (AEBSF), Aprotinin, bestatin, trans-epoxysuccinyl-L-leucyl-amido (4-guanidino) butane (E-64), Leupeptin and sodium EDTA.
The crude protein extracts were homogenised in a Waring commercial blendor for 5 minutes and filtered through Miracloth from Calbiochem. The three lichen crude extracts were freeze-dried overnight using the Virtis Freezemobile 25EL and stored at -20°C.

**Splat assay:**

11mg of freeze-dried crude extract *(Nephroma arcticum, Umbilicaria hyperborea* and *Platismatia glauca)* were resuspended in 500µl of ultra-pure water in Eppendorf tubes and centrifuged for 5 minutes at 13,000rpm in a microcentrifuge (MSE Micro Centaur). The supernatants were retained and 50µl aliquots of each extract were tested for heat stability for 2 minutes at 95°C in an Eppendorf Thermomixer 5436. Both crude extracts (heat-treated or not) were immediately assayed for AFP activity using a version of the splat assay as detailed below. (Further information about the test used is given in Byass et al., Unilever WO 98/04148).

The samples for Splat assay were mixed with an equal volume of 60% sucrose, giving a final concentration of 30% sucrose. Small aliquots (5µl) of the sample to be tested were pressed between two microscope cover slips. These were rapidly cooled by transfer into a bath of dry ice/trimethylpentane (-70°C), and were then transferred to a bath of trimethylpentane cooled to -6°C. AFP activity was assessed in semi-quantitative terms, according to the scoring system given below.

| Splat Score | Observed crystal morphologies |
|---|---|
| +++++ | Very small, very dense crystals. |
| ++++ | Small, not dense; or small, quite dense with some medium-sized crystals. |
| +++ Small, not dense with medium-large crystals; or small-medium, not dense. | |
| ++ Medium or large crystals with some small crystals | |
| + Large, round discrete crystals (e.g. 30% sucrose control). | |

| Results: | |
|---|---|
| **Test sample** | *SPLAT score* |
| *Nephroma arcticum* | +++++ |
| *Umbilicaria hyperborea* | ++ |
| *Platismatia glauca* | ++ |
| Negative control (30% sucrose only) | + |

The results show that out of the three lichens tested, Nephroma *arcticum* produces by far the most AFP activity.

**EXAMPLE 4**

Scaling-up the preparation of AFP-containing extracts from *Nephroma arcticum*.

8.94 grams of freeze-dried *Nephroma* arcticum extract (prepared as described in example 3 under "protein extraction") was resuspended in 70ml of ice cold pure water and stirred for 20 minutes at 4°C. The crude extract was centrifuged for 20min at 8000rpm in a Sorval centrifuge using a GSA rotor. The supernatant was filtered using Nalgene 0.8 µm membrane filtration sterilisation unit followed by 0.2 µm membrane filtration sterilisation unit. The brown extract of *Nephroma arcticum* was aliquotted in plastic tubes and stored frozen until required.

**EXAMPLE 5**

Investigation of heat stability of AFP extracted from *Nephroma arcticum*

If an AFP is to be used as a food ingredient in a foodstuff that is pasteurised during its manufacture, it is important that the AFP is stable to temperatures that are likely to be used. In this example, the heat stability of the AFP extracted from *Nephroma arcticum* is investigated.

The AFP-containing extract (prepared as in Example 3) was analysed for total protein concentration using the BCA protein assay reagent kit (supplied by Pierce). The extract was found to have a total protein content of approximately 5.8mg/ml. A small sample of this extract was diluted so that it had a final concentration of 2mg/ml protein and then 16µl of the diluted extract was placed into each of 5 Eppendorf tubes. Four of the tubes were placed in an Eppendorf Thermomixer at 95°C. Each tube was exposed to the heat treatment for a different length of time: either 1, 2, 5, or 10 minutes. The fifth tube was used as a control and was not exposed to the heat treatment. Following heating, the samples were immediately placed on ice and assayed for AFP activity using the splat assay.

| SPLAT activities on 2mg/ml Nephroma arcticum extract: | |
|---|---|
| **Material** | **SPLAT score** |
| *Nephroma arcticum* extract, not heated | +++++ |
| *Nephroma arcticum* extract, heated for 1 minute | +++++ |
| *Nephroma arcticum* extract, heated for 2 minutes | +++ |
| *Nephroma arcticum* extract, heated for 5 minutes | ++ |
| *Nephroma arcticum* extract, heated for 10 minutes | ++ |

As the AFP extracted from *Nephroma arcticum* can survive heating at 95°C, it is suitable for use in food-stuffs that are pasteurised during their manufacture. For example, an ice-cream "mix" would typically be pasteurised by holding at a temperature of 82°C for 25 seconds before cooling and freezing. The results show that the AFP extracted from *Nephroma arcticum* could survive such a pasteurisation process.

### EXAMPLE 6

Preparation of AFP from *Nephroma arcticum* by a process that is suitable (subject to toxicological testing) for inclusion in frozen foods.

*Sampling*

Two separate preparations were made in the same way, and the extracts were pooled together to.make the final extract. 29g (Extract 1) and 35g (Extract 2) of tissue were used respectively. The tissue used was *N.arcticum* gathered by one of the inventors (Rolv Lundheim) in Norway during the winter seasons of 1999/2000, shipped (on dry ice) to laboratories in the U.K. and stored at -70°C until use.

*Buffers used*

The Extraction buffer consisted of 200mM Tris-HCl (Fisher chemicals), 20mM Ascorbate (sodium ascorbate, Sigma), 10 mM EDTA (disodium salt, Sigma) at pH7.8.
The Dialysis buffer was 50mM Tris-HCl, 3mM EDTA at pH7.5.

*Extraction*

The *N.arcticum* tissue was ground to a fine powder in liquid nitrogen using a mortar and pestle. The ground material was placed in a beaker with 180ml of chilled extraction buffer and stirred on ice for 30 mins. The resulting extract was filtered through muslin. The solid material was reground using the pestle and mortar and a small volume of additional buffer (Extract 1, 40ml Extract 2, 80ml). This second extraction was also filtered through muslin. The combined filtrate from the two steps was the crude extract (Extract 1, -140ml Extract 2, ~200ml).

*Purification*

The crude extract was heated in a shaking water bath at 60°C.
When the temperature of the extract reached 60°C it was incubated for 15 minutes. The extract was then cooled on ice before centrifugation for 20 minutes at 15,000rpm in a Sorvall RC-5B with an SS34 rotor cooled to 4°C. As the supernatant was not fully clarified after this it was re-centrifuged in clean tubes at the same speed.
The extract was then dialysed using Spectra/Por dialysis tubing with a molecular weight cut off of 6,000-8,000D. The dialysis buffer was as described above. The buffer volume was 2L (Extract 1) or 3L (Extract 2), and 3 changes of buffer were carried out over a period of ~18 hrs. Dialysis was carried out at 4°C.

*Concentration*

The dialysed extract was retained in the dialysis tubing, and placed in a sandwich box. The sample was covered with PEG (polyethylene glycol 17, 500, Fluka). The sample was kept at 4°C while the volume reduced. After

~2 days in the PEG bath, the volume of the extracts were 35ml (Extract 1) and 30ml (Extract 2). After concentration, the extracts were again centrifuged at 15,000 rpm and filtered through 0.45 and then 0.22μm syringe filter units.

Preparation and characterisation of Final Extract

The two extracts prepared as described above were pooled together and aliquoted into 5ml amounts before freezing at -20°C. This was the extract provided for RI assay and water ice tests (refer to example 7).

The extract was tested for AFP activity using the splat assay. The following results were obtained (scored as described in example 2). The results show that the final extract can be diluted 100-fold and still show activity in the splat assay.

| Dilution | Splat score |
|----------|-------------|
| 1/2 | +++++ |
| 1/10 | ++++/+++++ |
| 1/50 | ++++ |
| 1/100 | ++/+++ |

The pH of the extract as measured was pH 6.9.

The protein concentration of the extract was estimated using the Coomassie Plus-200 protein assay from Pierce using the standard protocol microwell plate version according to the manufacturer's instructions. The total protein concentration of the final extract was estimated to be ~1mg/ml.

## EXAMPLE 7

Determination of how much AFP to add to a food-stuff

*Re-crystallisation Inhibition (RI) assay:*

A solution containing AFP from *Nephroma arcticum* (as prepared in example 6) was adjusted to a sucrose level of 30 wt% with sucrose crystals. A 3 μl drop of the sample was placed on a 22 mm coverslip.

A 16 mm diameter cover-slip was then placed on top and a 200 g weight was placed on the sample to ensure a uniform slide thickness. The edges of the coverslip were sealed with clear nail varnish. The slide was placed on a Linkam THM 600 temperature controlled microscope stage. The stage was cooled rapidly (50°C per minute) to -40°C to produce a large population of small crystals. The stage temperature was then raised rapidly (50°C per minute) to -6.0 °C and held at this temperature. The ice-phase was observed at -6.0 °C using a Leitz Ortholux II microscope. Polarised light conditions in conjunction with a lambda plate were used to enhance the contrast of the ice crystals. Images were recorded at T=0 and T=60 minutes and analysed using image analysis software (AnalySIS, Soft-Imaging Software GmbH, D48153, Munster, Hammerstrasse, 89 Germany). The crystal growth was calculated as follows.

The mean size of a population of ice crystals at a particular time was determined by capturing an image of the population (using the equipment mentioned above) and then selecting a region of the image that contained at least 200 ice crystals. The longest diameter of each crystal was determined by drawing around them and calculating the longest diameter using the image analysis software.

Each RI experiment was carried out 3 times (therefore the images of at least 600 ice crystals were analysed). The data in the table record the mean ice crystal growth (i.e. the difference in the mean ice crystal diameter at time zero and after 60 minutes). Confidence limits were calculated for a confidence level in the mean of 95%.

Several dilutions of a preparation of *Nephroma arcticum* AFP were made in order to find out how far the sample could be diluted and still achieve an effective inhibition of re-crystallisation of ice. In practical terms we consider an AFP preparation or a dilution of an AFP preparation to achieve an effective inhibition of re-crystallisation of ice if a sample of said dilution or preparation results in ice crystal growth in the RI assay of 3μm or less.

*RI assay results*

Mean crystal growth was determined by analyzing the images of 1200 crystals.

| Dilution of AFP preparation | Crystal growth (mean in μm) | Confidence limits |
|---|---|---|
| 1 in 10 | 1.09 | +/- 0.17 |
| 1 in 20 | 2.01 | +/- 0.25 |
| 1 in 50 | 3.67 | +/- 0.48 |
| 1 in 100 | 4.79 | +/- 0.62 |
| 1 in 200 | 6.84 | +/- 0.62 |

The results show that the AFP preparation can be diluted 20-fold and still an effective inhibition of the re-crystallisation of ice is achieved. Therefore, a 1 in 20 dilution (= 5%) of this preparation was used in frozen food prototypes as shown in Example 8.

**EXAMPLE 8**

Application of lichen AFP in a water-ice

Two liquid pre-mixes were made for the preparation of water-ice:

(A) 20% sucrose + 0.2% locust bean gum in water (negative control; not according to the invention).
20% sucrose + 0.2% locust bean in water containing AFP from *Nephroma arcticum*. The AFP had been diluted in the pre-mix to a concentration that would give an RI value of approximately 2μm. In this instance this was a dilution of 1 in 20 or 5% - see example 7 for RI data.

Therefore, pre-mix B had the following composition:

| Ingredient | % by weight |
|---|---|
| Sucrose | 20.00 |
| LBG | 0.2 |
| AFP preparation (in aqueous buffer) | 5.00 |
| Water | 74.80 |

*Determination of hardness by The Vickers Hardness test*

Cuboid bars of water ice (10mm x 55mm x 95mm) were prepared for each composition (A, and B) as follows. Each composition was frozen to -3.5°C in a Gelato Chef 2000 (Magimix UK Ltd) Ice Cream maker. The mixture was transferred to silicone rubber moulds with internal dimensions of 10mm x 55mm x 95mm, previously cooled to -30°C. The preparation was then cooled at -30°C for 1 hour before de-moulding and storing at -25°C prior to hardness testing, using the Vickers Hardness test.

The Vickers Hardness test is an indentation test that involves pushing a pyramid shaped indentor into the surface of material and recording the force applied as a function of tip displacement. Force and displacement are measured during the indentation loading cycle and the unloading cycle. The test is described in "Handbook of Plastics Test materials" Ed. R.P. Brown, Pub. George Godwin Limited, The Builder Group, 1-3 Pemberton Row, Fleet Street, London, 1981. The Vickers pyramid geometry is an engineering industry standard (BSi 427, 1990). It has an apex angle at the tip of 136°. Hardness is determined as:

$$H_V = \frac{F_{max}}{A}$$

where $H_v$ is the Vickers Hardness, $F_{max}$ is the maximum applied force and $A$ is the projected area of the indentation left in the material's surface. The area A is determined by assuming the indentation has the same geometry as the indentor that formed it, i.e. a Vickers pyramid, and therefore the projected area can be determined from the indent depth given by $d_i$.

$$A = 24.5d_i^2$$

*Results from hardness testing*

Three frozen blocks were made from each mix. A total of eight hardness readings were made for each frozen mixture. [The 8 indentations were distributed between the 3 blocks so as to accommodate any variation from one block to another in the results].

| Hardness data (Mpa) | | |
| --- | --- | --- |
| Frozen mixture | Hardness (mean of 8 readings) | Standard deviation |
| Sucrose + LBG | 1.03 | 0.32 |
| Sucrose + LBG + AFP | 2.75 | 0.78 |

The results show that the lichen AFP can modify the texture of a frozen food significantly. In this particular formulation (which is typical of a water ice) and using this particular concentration of AFP, a harder texture is produced.

**EXAMPLE 9**

Purification of AFP for N-terminal analysis

To achieve accurate N-terminal sequence data, a very pure sample needs to be prepared. A method for preparing such a sample is given below. It is important to note that it would not normally be necessary to prepare such a pure sample for use as a food additive.

*Extraction of AFP*

8.6 gram of clean, dry lichen tissue was ground to a fine powder in liquid nitrogen in a pre-cooled pestle and mortar. The powder was homogenised in a 1:1 ratio in buffer A (0.2 M Tris; 10 mM EDTA; 20 mM ascorbate; pH7.8). The homogenate was placed in a 50 ml Falcon tube and sonicated on ice for 15 min prior to transfer to 1.5 ml Eppendorf tubes and centrifugation for 4 min; 14 000 rpm; 4°C. The supernatant was collected and kept on ice. The pellets were re-extracted in 4 ml of buffer A and the supernatant was added to the supernatant obtained earlier. The pooled supernatant was clarified by centrifugation for 10 minutes; 14 000 rpm; 4°C and desalted on a HiTrap desalting column (Pharmacia) according to the manufacturer's instructions into buffer B (50 mM TrisHCl pH 7.5).

*Ion-exchange chromatography*

The desalted sample was filtered (0.2 µm microfilter) and 1.5 ml was applied to a 6 ml Mono Q anion exchange column pre-equilibrated in the same buffer at a flow rate of 1 ml/min. The eluate was monitored for conductivity and OD 280 and 2 ml fractions were collected. When the OD 280 returned to base line a linear gradient of 0 - 0.5 M NaCl was applied. The antifreeze activity eluted at about 0.2 M NaCl (as adjudged by the Splat assay).

*Affinity chromatography*

Active anion exchange fractions were pooled and concentrated (by dialysis in polethylene glycol compound "PEG" at 4°C). The concentrated sample was applied to a 28 ml Concanavalin A (ConA) column (Pharmacia FPLC), which had been equilibrated in 3 column volumes of buffer C [Tris buffered saline (TBS - 20 mM Tris/HCl; 0.5 M NaCl; pH 7.4); 1 mM CaCl$_2$; 1 mM MnCl$_2$] and washed through in 2 column volumes of buffer C. The activity was eluted with approximately 3 column volumes of buffer D (TBS, 100 mM methyl $\alpha$-D-mannopyranoside). The flow rate was 2 ml min$^{-1}$ and 2 ml fractions were collected throughout. The active fractions were pooled and concentrated and applied to a gel filtration column as described below.

*Gel filtration chromatography*

The active fractions were concentrated 10 fold in Millipore 30 kDa cut off concentrators and the pass and retentate tested for antifreeze activity. The active concentrates were pooled and further concentrated (10 fold); 22 µl was filtered and loaded onto a Superdex 75 gel permeation column on the Pharmacia SMART system which had been pre-equilibrated in buffer B (50 mM Tris/HCl pH 7.5). The flow rate was 40 µl/min and 50 µl fractions were collected. The active fractions after the gel filtration step were pooled and concentrated and used for N-terminal analysis as described in example 10.

*Conclusion*

During the purification protocol given above, gel electrophoresis (SDS-PAGE with silver staining) was used to identify the AFP. This technique consistently identified a negatively staining band at 29kDa that co-purified with AFP activity (as adjudged by the Splat assay). Therefore, this protein was known to be the AFP and it was this band that was N-terminal sequenced as described in example 10.

## EXAMPLE 10

Determination of the N-terminal sequence of the AFP derived *from Nephroma arcticum.*

90 µl purified *N. arcticum* AFP sample (prepared as described in example 9) was applied equally to four adjacent lanes and separated by SDS-PAGE prior to western blotting onto PVDF membrane. The membrane had been soaked in methanol and the blotting buffer used was 10 mM CAPS, pH11 plus 10 % methanol. The membrane was stained with Ponceau stain and the relevant bands marked with a needle before removal of the stain with water. The membrane was airdried and stored. The sequencing was carried out using a Procise® 492 protein sequencer (Applied Biosystems) according to the manufacturer's instructions. The N-terminal sequence obtained is given below. The inventors believe this sequence to be novel and to be unique to the AFP that is the subject of this invention

| *N-terminal sequence as described in example 10* [SEQ. ID no 1] | |
| --- | --- |
| 1 L | 10 F |
| 2 V | 11 G |
| 3 I | 12 V |
| 4 G | 13 V (S) |
| 5 S | 14 A |
| 6 T | 15 A |
| 7 A | 16 A |
| 8 Q (E) | 17 T |
| 9 N | |

## EXAMPLE 11

Production of AFP in a Genetically Modified Organism

In order to produce the *Nehphroma arcticum* AFP in a more commercially convenient host organism, the AFP gene is cloned and expressed in the chosen host system. GM-expression systems based on bacteria, yeasts, plants, plant cells, insect cells, and animal cells are known; those based on bacteria, yeasts, and plants are generally preferred. It is taught how to clone the AFP gene from *Nephroma arcticum* below.

*Cloning the AFP gene from Nephroma arcticum*

The N-terminus of the *Nephroma arcticum* AFP is given in example 10. Once this sequence is known, the entire gene for the AFP can be cloned - without making any further inventive steps - by using standard techniques for cloning eukaryotic genes that are well known in The Art. A description of this procedure is given below.
For eukaryotes, there are several approaches that can be taken to clone genes for which the N-terminal amino acid sequence is known.
All eukaryotic genes have a poly A tail and this information is used for cloning. A typical first step would be to obtain a sample *of Nephroma arcticum* that has been exposed to cold weather for a prolonged period (and therefore has been induced to make AFP). This is followed by the isolation of poly A RNA from said sample by using standard reagent kits that are sold for this purpose and following the manufacturer's instructions. Alternatively, the poly A RNA could be isolated from an AFP-producing culture of a component organism of the lichen. In this case, the cultured cells would be cold-shocked to induce the cells to make AFP. This can be achieved by placing a flask of the cells on ice. Once poly A RNA has been isolated, complementary DNA can be made using the enzyme reverse transcriptase. Standard kits are available for this step. First strand cDNA can be used directly in the Polymerase Chain Reaction (PCR) or double strand DNA made and used in PCR or to make a cDNA library.

In PCR, the DNA is used as a template in the reaction. The PCR reaction requires two primers. One primer is designed to the known N-terminal amino acid sequence and is most likely to be degenerate to take into account the degeneracy inherent in codon usage. An example of a primer sequence to the N-terminal amino acid sequence T A Q N F G V V A is:

```
ACI GCI CAR AAY TTY GGI GTI GTI GC          [SEQ. ID no 2]
```

where I = inosine, R = A or G, Y = C or T

In addition to this example, a person skilled in the art could design many other primers that could be designed to different regions of the N-terminal amino acid sequence (as given in example 10). Furthermore persons skilled in the art often prefer to use a number of primers to the N-terminal sequence in a cloning experiment.

The second primer used is a dT oligonucleotide sequence which will bind to the poly A tail at the 3' end of the clone. The PCR reaction using these primers will result in a band which can be isolated and cloned into a suitable vector using standard methodologies. The vector insert is then sequenced and the DNA translated to its corresponding amino acid sequence to show the presence of the N-terminal sequence obtained from *Nephroma arcticum*. By cloning the DNA sequence into an expression vector, AFP can be produced and tested to show that it has the same properties as AFP isolated directly from *Nephroma.*

An alternative cloning route is to use the cDNA to make a library in a suitable vector. This library can then be screened for the gene encoding the *Nephroma* protein. Several routes can be used to screen for the gene. For example, a degenerate primer can be made to the N-terminal amino acid sequence as described above and labelled in some way, such as using radioactivity. This primer can then be used to screen the library since it will bind to its complementary strand present in the library. The identified clone can then be sequenced as described above. An antibody can also be made to the purified AFP protein from *Nephroma* and used to screen the library if it has been made in an expression vector. Again, the clone can be sequenced as described above.

**EXAMPLE 12**

AFP production by culturing organisms obtained from *Nephroma arcticum*

Lichen fungi can be grown separately under laboratory conditions. This requires a growth medium that supplies the nutrients that the fungus normally gets from the photobiont, and also trace elements that the lichen gets from its environment. The fungus can either be grown in liquid media or on the surface of agar containing the appropriate growth media. The fungus can also be grown on sterilised soil (Stocker-Worgotter, E. and Turk R. 1991. Lichenologist. 23. 127-138).

Ascocarps (the sexual fruiting body of ascomycete fungi) are cut from the lichen thalli and washed in phosphate-buffered saline as described by Bubrick and Galun (1986. Lichenologist 18, 47-49). The ascocarps can then be fixed to the top of Petri-dishes containing a suitable growth medium (Bishcoff, H.W. and Bold H.C. 1963. University of Texas Publications No. 6318, Physiological studies. 4, 1-95). The spores will be released and fall onto the growth medium. After the spores germinate, small bocks of agar can be transferred to liquid media (Ahmadjian, V. 1993. The Lichen symbiosis. John Wiley & sons Inc. New York.) Examples of growth media are given by Stocker-Worgotter, E. and Turk R. 1991. (Lichenologist. 23. 127-138).

Cultures of the fungi can also be obtained from fragmented thalli as described by Yamamoto Y (1990. PhD dissertation. Kyoto University) and modified by Stocker-Worgotter (1995. Canadian Journal of Botany 74 (Suppl 1) S579-S589.

The photobiont of lichen can be cultured in a similar way. To obtain axenic cultures, small thalli fragments are scratched and algal cells are removed under a dissecting microscope by means of a micropipette and transferred to a suitable growth medium (Ahmadjian, V. 1973. In: The Lichens V. Ahmadjian and M.E. Hale, eds. Academic Press, New York, pp. 653-659). The algal cells can grow on both liquid and solid (agar) media. For a detailed description of culture methods and media see Ahmadjian 1993. Nephroma arcticum comprises an algae of the genus Coccomyxa (Peveling, E. and Galun, M. 1976. New Phytol. 77. 713) and a cyanobacterium of the genus *Nostoc* (Wetmore, C.M. 1960. Publ. Mus.

Mich. State. Univ. Biol. Ser 1, 369). Cyanobacteria of the genus *Nostoc* and algae of the genus *Coccomyxa* can be obtained from culture collections such as the American Type Culture Collection. These organisms did not originate from *Nehproma arcticum,* but work performed on these cultures has provided information on suitable growth media for culturing such organisms. Cyanobacteria of the genus *Nostoc* and that were obtained from lichen have been cultivated by several investigators (Ahmadjian 1993).

**Claims**

1. An antifreeze protein having a molecular weight of between 25 and 33 kDa which is capable of being derived from the lichen *Nephroma arcticum*, said antifreeze protein having an amino acid sequence part of which shows at least 80% overlap with the sequence L-V-I-G-S-T-A-Q(E)-N-F-G-V-V(S)-A-A-A-T.

2. An antifreeze protein according to claim 1, said protein havin an amino acid sequence part of which shows at least 90% overlap with the sequence L-V-I-G-S-T-A-Q(E)-N-F-G-V-V(S)-A-A-A-T.

3. An antifreeze protein according to any of claims 1-2, said protein having an amino acid sequence comprising the sequence L-V-I-G-S-T-A-Q(E)-N-F-G-V-V(S)-A-A-A-T.

4. An antifreeze protein according to any of claims 1-3, said protein having at least one covalent modification.

5. An antifreeze protein according to claim 4 wherein the modification is glycosylation.

6. A nucleic sequence encoding an antifreeze protein according to any of the preceding claims.

7. A vector comprising a nucleic acid sequence according to claim 6.

8. A method for producing an antifreeze protein (AFP) according to any of claims 1 to 5, said method comprising the steps of:

    (i) harvesting *Nephroma arcticum* from the wild;
    (ii) preparing a protein-containing extract from the material of step (i), said extract exhibiting AFP activity.

9. A method for producing an antifreeze protein (AFP) according t any of claims 1 to 5, said method comprising the steps of:

(i) culturing an organism containing a nucleic acid sequence accordin to claim 6, under the control of suitable gene regulatory elements and under conditions in which the protein is synthesised, and
(ii) recovering from the culture an extract that exhibits AFP activity.

10. A method according to claim 9, wherein the organism is one of the constituent organisms of *Nephroma arcticum*.

11. A method according to claim 9, wherein the organism contains the nucleic acid sequence according to claim 6 as a consequence of genetic modification.

12. A method according to claim 11, wherein the antifreeze protein is directed to be secreted into the culture medium.

13. A genetically modified non-human organism, containing a nucleic acid sequence according to claim 6.

14. A protein-containing extract exhibiting AFP activity, prepared by any of the methods of claims 8 to 12, said extract being suitable for use as a food additive.

15. A food product containing an antifreeze protein according to any of claims 1-5.

16. A food product according to claim 15, said food product being a frozen confectionery product.

**Patentansprüche**

1. Gefrierschutzprotein mit einem Molekulargewicht zwischen 25 und 33 kDa, das von der Flechte *Nephroma arcticum* gewonnen werden kann, wobei das Gefrierschutzprotein eine Aminosäuresequenz aufweist, von der ein Teil eine mindestens 80%ige Überlappung mit der Sequenz L-V-I-G-S-T-A-Q(E)-N-F-G-V-V(S)-A-A-A-T zeigt.

2. Gefrierschutzprotein nach Anspruch 1, wobei das Protein eine Aminosäuresequenz aufweist, von der ein Teil eine mindestens 90%ige Überlappung mit der Sequenz L-V-I-G-S-T-A-Q(E)-N-F-G-V-V(S)-A-A-A-T zeigt.

3. Gefrierschutzprotein nach einem der Ansprüche 1 - 2, wobei das Protein eine Aminosäuresequenz aufweist, umfassend die Sequenz L-V-I-G-S-T-A-Q(E)-N-F-G-V-V(S)-A-A-A-T.

4. Gefrierschutzprotein nach einem der Ansprüche 1 - 3, wobei das Protein mindestens eine kovalente Modifikation aufweist.

5. Gefrierschutzprotein nach Anspruch 4, worin die Modifikation eine Glykosylierung ist.

6. Nukleinsäuresequenz, kodierend ein Gefrierschutzprotein nach einem der vorhergehenden Ansprüche.

7. Vektor, umfassend eine Nukleinsäuresequenz nach Anspruch 6.

8. Verfahren zur Herstellung eines Gefrierschutzproteins (GSP) nach einem der Ansprüche 1 - 5, wobei das Verfahren die folgenden Schritte umfasst:

(i) Ernten von *Nephroma arcticum* aus der Wildnis;
(ii) Herstellen eines proteinenthaltenden Extrakts aus dem Material von Schritt (i), wobei das Extrakt GSP-Aktivität zeigt.

9. Verfahren zur Herstellung eines Gefrierschutzproteins (GSP) nach einem der Ansprüche 1 - 5, wobei das Verfahren die folgenden Schritte umfasst:

(i) Züchten eines Organismus, enthaltend eine Nukleinsäuresequenz nach Anspruch 6, unter der Kontrolle geeigneter genregulierender Elemente und unter Bedingungen, unter denen das Protein synthetisiert wird, und
(ii) Gewinnen eines Extrakts von der Kultur, das GSP-Aktivität zeigt.

10. Verfahren nach Anspruch 9, worin der Organismus einer der konstituierenden Organismen von *Nephroma arcticum* ist.

11. Verfahren nach Anspruch 9, worin der Organismus die Nukleinsäuresequenz nach Anspruch 6 als eine Folge genetischer Veränderung enthält.

12. Verfahren nach Anspruch 11, worin das Gefrierschutzprotein in das Kulturmedium abgeschieden werden soll.

13. Genetisch veränderter, nicht menschlicher Organismus, enthaltend eine Nukleinsäuresequenz nach Anspruch 6.

14. Proteinenthaltender Extrakt, der GSP-Aktivität zeigt, hergestellt nach einem der Verfahren der Ansprüche 8 - 12, wobei der Extrakt zur Verwendung als ein Nahrungsmittelzusatz geeignet ist.

15. Nahrungsmittelprodukt, enthaltend ein Gefrierschutzprotein nach einem der Ansprüche 1 - 5.

16. Nahrungsmittelprodukt nach Anspruch 15, wobei das Nahrungsmittelprodukt ein gefrorenes Konfektprodukt ist.

**Revendications**

1. Une protéine antigel ayant un poids moléculaire entre 25 et 33 kDa qui peut être obtenue à partir du lichen *Nephroma arcticum*, ladite protéine antigel ayant une partie de séquence d'acide aminé qui montre au moins 80 % de recouvrement avec la séquence L-V-I-G-S-T-A-Q(E)-N-F-G-V-V(S)-A-A-A-T.

2. Une protéine antigel selon la revendication 1, dans laquelle la protéine a une partie de la séquence d'amino acide qui montre au moins 90 % de recouvrement avec la séquence L-V-I-G-S-T-A-Q(E)-N-F-G-V-V-(S)-A-A-A-T.

3. Une protéine antigel selon l'une des revendications 1 et 2, dans laquelle ladite protéine a une séquence d'amino acide qui comprend la séquence L-V-I-G-S-T-A-Q(E)-N-F-G-V-V(S)-A-A-A-T.

4. Une protéine antigel selon l'une des revendications 1 à 3, dans laquelle ladite protéine a au moins une modification covalente.

5. Une protéine antigel selon la revendication 4, dans laquelle la modification est une glycosylation.

6. Une séquence nucléique codant une protéine antigel selon l'une des revendications précédentes.

7. Un vecteur comprenant une séquence d'acide nucléique selon la revendication 6.

8. Un procédé pour produire une protéine antigel (AFP) selon l'une des revendications 1 à 5, ledit procédé comprenant les étapes de :

   (i) recueillir *Nephroma arcticum* dans la nature,
   (ii) préparer un extrait protéique de la matière de l'étape (i), ledit extrait ayant une activité AFP.

9. Un procédé pour produire une protéine antigel (AFP) selon l'une des revendications 1 à 5, méthode comprenant les étapes de :

   (i) cultiver un organisme contenant une séquence d'acide nucléique selon la revendication 6, sous le contrôle d'éléments de régulation des gènes appropriés et dans des conditions dans laquelle la protéine est synthétisée et
   (ii) prélever de la culture un extrait qui présente une activité AFP.

10. Un procédé selon la revendication 9, dans lequel l'organisme est l'un des organismes constituant le *Nephroma arcticum*.

11. Un procédé selon la revendication 9, dans lequel l'organisme contient la séquence d'acide nucléique selon la revendication 6 comme conséquence d'une modification génétique.

12. Un procédé selon la revendication 11, dans lequel la protéine antigel est dirigée pour être sécrété dans le milieu de culture.

13. Un organisme génétiquement modifié non humain contenant une séquence d'acide nucléique selon la revendication 6.

14. Un extrait contenant une protéine qui présente une activité AFP préparé par l'un des procédés selon les revendications 8 à 12, ledit extrait étant utilisable pour l'utilisation comme additif alimentaire.

15. Un produit alimentaire contenant une protéine antigel selon l'une des revendications 1 à 5.

16. Un produit alimentaire selon la revendication 15, ledit produit alimentaire étant un produit gelé.

SEQUENCE LISTING

<110> Unilever plc
    Unilever NV

<120> Anti-freeze proteins, their production and use

<130> F3258

<160> 2

<170> PatentIn version 3.0

<210> 1

  <211> 17
  <212> PRT
  <213> Nephroma arcticum

<220>

  <221> VARIANT
  <222> (13)..(13)
  <223> RESIDUE 13 MAY BE V OR S

<400> 1

```
Leu Val Ile Gly Ser Thr Ala Glx Asn Phe Gly Val Val Ala Ala
Ala
1               5                   10                  15

Thr
```

<210> 2

  <211> 26
  <212> DNA
  <213> Artificial

<220>

  <223> PCR primer

<220>

  <221> modified_base
  <222> (3)..(3)
  <223> i

```
<220>

    <221> modified_base
    <222> (6)..(6)
    <223> i

<220>

    <221> modified_base
    <222> (18)..(18)
    <223> i

<220>

    <221> modified_base
    <222> (21)..(21)
    <223> i

<220>

    <221> modified_base
    <222> (24)..(24)
    <223> i

<400> 2
```

```
acngcncara ayttyggngt ngtngc
26
```